(19)

Europäisches
Patentamt

European
Patent Office

Office européen
des brevets

(11) **EP 3 325 970 B1**

(12) **EUROPEAN PATENT SPECIFICATION**

(45) Date of publication and mention
of the grant of the patent:
**16.09.2020  Bulletin 2020/38**

(21) Application number: **16745594.8**

(22) Date of filing: **20.07.2016**

(51) Int Cl.:
*G01N 33/569* [(2006.01)]   *G01N 33/58* [(2006.01)]

(86) International application number:
**PCT/US2016/043129**

(87) International publication number:
**WO 2017/019404 (02.02.2017 Gazette 2017/05)**

(54) **HIGH THROUGHPUT METHODS FOR VIRUS QUANTIFICATION**

HOCHDURCHSATZVERFAHREN ZUR VIRUSQUANTIFIZIERUNG

PROCÉDÉS DE QUANTIFICATION DE VIRUS À HAUT RENDEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.07.2015  US 201562196886 P**

(43) Date of publication of application:
**30.05.2018  Bulletin 2018/22**

(73) Proprietor: **Boehringer Ingelheim Animal Health
USA Inc.
Duluth, GA 30096 (US)**

(72) Inventor: **REYES, Jean
69003 Lyon (FR)**

(74) Representative: **D Young & Co LLP
120 Holborn
London EC1N 2DY (GB)**

(56) References cited:
**WO-A1-98/01582**

• **ERIKA HAMMARLUND ET AL: "A Flow
Cytometry-Based Assay for Quantifying
Non-Plaque Forming Strains of Yellow Fever
Virus", PLOS ONE, vol. 7, no. 9, 19 September
2012 (2012-09-19), page e41707, XP55524798,
DOI: 10.1371/journal.pone.0041707**
• **Erika Hammarlund ET AL: "A Flow
Cytometry-Based Assay for Quantifying
Non-Plaque Forming Strains of Yellow Fever
Virus", PLoS ONE, vol. 7, no. 9, 19 September
2012 (2012-09-19), page e41707, XP055524798,
DOI: 10.1371/journal.pone.0041707**

Printed by Jouve, 75001 PARIS (FR)

## Description

### CROSS-REFERENCE TO RELATED APPLICATIONS

This application claims priority to U.S. provisional application 62/196,886 filed on July 24, 2015.

### FIELD OF THE INVENTION

This invention relates to high throughput methods of determining a viral titer. The instant invention addresses the need for a more rapid and cost effective method of quantitating infectious viral particles in a sample.

### BACKGROUND OF THE INVENTION

A particular challenge in the delivery of a gene by a viral vector or a virus for therapeutic purposes is the preparation and accurate quantification of clinical dosage forms. The production of viral vaccines, recombinant proteins using viral vectors and viral antigens all require virus quantification to continually adapt and monitor the process in order to optimize production yields and respond to the ever changing demands and applications.

Virus titer determination or virus quantification involves counting the number of viruses in a specific volume to determine the virus concentration. Traditional methods include viral plaque assays which determine the number of plaque forming units (pfu) in a virus sample and the Tissue Culture Infective Dose ($TCID_{50}$) or Fluorescence Active Infectious Dose (FAID50) which measures the infectious virus titer. This $TCID_{50}$ assay quantifies the amount of virus required to kill 50% of infected hosts or to produce a cytopathic effect in 50% of inoculated tissue culture cells. The traditional methods are generally slow and labor-intensive, and suffer from limitations including a high degree of inter-assay variability.

Enzyme-Linked Immunosorbent Assay (ELISA) is a more modem variation of a protein assay that utilizes a specific antibody linked to an enzyme to detect the presence of an unknown amount of antigen (i.e. virus) in a sample. The antibody-antigen binding event is detected and/or quantified through the enzyme's ability to convert a reagent to a detectable signal that can be used to calculate the concentration of the antigen in the sample. The plate assays of the virus titer determination that are based on immunofluorescence detection using an ELISA are developed, however they are used to quantify proteins from virus samples and not to quantify infectious viruses.

Flow cytometry or FACS (fluorescence-activated cell sorter) assays have been used to measure the number of infected cells in cell cultures infected at relatively high multiplicities of infection. For example, US6,248,514 discloses the use of flow cytometry to analyze cells infected using specified ranges of viral particle concentration and adsorption time yields. US7,476,507 discloses FACS-based methods for the determination of the viral titer of a culture of host animal host cells infected with a circovirus. However, for many applications, the cost, size and complexity of the flow cytometry instruments prevent wider use. International application WO98/01582 discloses a method of virus quantification but a single benchtop instrument which combines the functions of a digital microscope, an image cytometer and a cell counter is not disclosed.

ERIKA HAMMARLUND ET AL: "A Flow Cytometry-Based Assay for Quantifying Non-Plaque Forming Strains of Yellow Fever Virus", PLOS ONE, vol. 7, no. 9, 19 September 2012, page e41707 discloses a focus forming assay for determining a viral titer.

Recently, personal image cytometers are developed which combine the functions of a digital microscope, an image cytometer, and a cell counter in a single benchtop instrument. The commercially available image cytometers include CellInsight (ThermoFisher) and Cytell Cell Imaging System (GE Healthcare). These image cytometers are used to make individual cell, subcellular and multi-cellular measurements, and provide the opportunity for scientists to capture cellular and subcellular image data and analyze results with ease.

The instant invention addresses the need for an accurate and high throughput method of quantitating infectious viral particles in a sample.

### SUMMARY OF THE INVENTION

In one aspect, the invention provides A method of virus quantification comprising the steps of: providing a sample containing a virus; preparing serial dilutions of the sample; infecting host cells with the virus and incubating the culture; reacting an antigen expressed by the virus in infected cells with an antibody labeled with a fluorescent tag; determining the number of infected cells by using an image cytometer wherein the image cytometer is a component of a single benchtop instrument which combines the functions of a digital microscope, said image cytometer and a cell counter, preferably wherein said image cytometer is capable of making individual cell, sub-cellular and multi-cellular measurements and capturing cellular and sub-cellular image data; and determining the virus titer in the sample, wherein a straight line is plotted using linear regression between number of fluorescence tagged cells and virus titer using a virus reference.

As described herein are high throughput methods for the rapid determination and quantification of the infectious virus produced in a sample. Described herein are high throughput methods for the rapid determination and quantification of the infectious virus produced in a batch culture such that an optimum harvesting point can be obtained. The methods encompass providing a sample containing a virus, preparing serial dilutions of the sample, infecting host cells therewith and incubating the culture at an appropriate temperature, reacting an antigen expressed by the virus in infected cells with an antibody labeled with a fluorescent tag, determining the number of fluorescence tagged cells, thereby determining the virus titer in the sample. The high throughput methods may be carried out in a multiple-well plate format. In one embodiment, CellInsight (ThermoFisher) is used.

In one aspect of the embodiment, a straight line is plotted using linear regression between number of fluorescence cells and virus titer using a virus reference.

In another aspect of the embodiment, at least one of the serial dilutions of the sample falls within the range of the liner regression line.

In yet another aspect of the embodiment, the multi-well plates are used.

In yet another aspect of the embodiment, the number of infected cells in each well is determined by an image cytometer, such as CellInsight.

The virus can be wild-type virus, attenuated virus, or recombinant virus. When the virus is a recombinant virus, the antigen can be encoded by an exogenous gene. Typically the antigen reacts with at least one antibody, although the antibody can be a mixture of antibodies. The antibody can be polyclonal or monoclonal.

The sample can be a sample collected from fields, sample from optimization process, seed sample, main culture sample, active ingredient, or final product.

These and other embodiments are disclosed or are obvious from and encompassed by, the following Detailed Description.

## BRIEF DESCRIPTION OF DRAWINGS

The following detailed description may best be understood in conjunction with the accompanying drawings, incorporated herein by reference, in which:

Figure 1 illustrates a sample of dilutions.

Figure 2 illustrates a sample of plate design in a conventional plate assay.

Figure 3 illustrates a sample of plate design in high throughput virus titration.

Figure 4 illustrates a sample of plate design in high throughput virus titration.

Figure 5 depicts the linear regression of CPV2.

Figure 6 depicts the comparison between conventional virus titer method and high throughput virus titer method for CPV2.

Figure 7 depicts the linear regression of Pox virus (Avian ALVAC) expressing an equine influenza antigen.

Figure 8 depicts the comparison between conventional virus titer method and high throughput virus titer method for Pox virus (Avian ALVAC) expressing an equine influenza antigen.

## DETAILED DESCRIPTION

It is noted that in this disclosure and particularly in the claims, terms such as "comprises", "comprised", "comprising" can have the meaning attributed to it in U. S. Patent law; e.g., they can mean "includes", "included", "including" and that terms such as "consisting essentially of and "consists essentially of have the meaning ascribed to them in U.S. Patent law, e.g., they allow for elements not explicitly recited, but exclude elements that are found in the prior art or that affect a basic or novel characteristic of the invention.

The singular terms "a," "an," and "the" include plural referents unless context clearly indicates otherwise. Similarly, the word "or" is intended to include "and" unless the context clearly indicate otherwise. The word "or" means any one member of a particular list and also includes any combination of members of that list.

The term "imaging cytometer" or "Cell imaging system" as used herein refers to any device that will irradiate a particle suspended in a fluid medium with light at a wavelength, and is capable of detecting a light at the same or a different wavelength, wherein the detected light indicates the presence of a cell or an indicator thereon, and take images of the number of cells using optical microscopy. The indicator on the cell surface may be an antibody coupled to a fluorophore such as FITC, Hoechst stain, DAPI, GFP, TRITC and Cy5. The "imaging cytometer" or "image cytometer" may be coupled to a data visualization, data storage, and data management system for real-time analysis.

The term "host cell" as used herein refers to an isolated cell that is a host for the infection and replication of a virus. A "host cell" denotes a prokaryotic or eukaryotic cell. The "host cell" may be genetically altered, or is capable of being genetically altered by administration of an exogenous polynucleotide, such as a recombinant plasmid or vector. The "host cell" may be a cell established in stable lineage without any genetic modifications, only by repeated culture passages. The "host cell" may be primary cells that are neither modified nor stable. When referring to genetically altered cells, the term refers both to the originally altered cell and to the progeny thereof.

The term "in-process" refers to the monitoring of parameters that are characteristic of cell and virus culture, including a virally infected cell culture, throughout the period of the culture.

The monitoring can be continuous, such as monitoring the pH or oxygen content of the culture medium, or can be periodic monitoring wherein samples are withdrawn from the culture at selected time points, parameters such as viability or viral antigen content are detected and measured and the parameters are plotted versus the time of the culture.

The term "seed culture" or "seed" as used herein refers to a culture of host cells infected with a selected virus and which is then incubated for a period to allow the titer of virus to increase. Typically, but not necessarily, the volume of a seed culture is less than the volume of the subsequent main culture or fermentation medium that receives the seed culture.

The term "animal" is used herein to include all mammals, birds and fish. The animal as used herein may be selected from the group consisting of equine (e.g., horse), canine (e.g., dogs, wolves, foxes, coyotes, jackals), feline (e.g., lions, tigers, domestic cats, wild cats, other big cats, and other felines including cheetahs and lynx), bovine (e.g., cattle, buffalos), swine (e.g., pig), ovine (e.g., sheep), caprine (e.g., goats), camelids (e.g., lamas), avian (e.g., chicken, duck, goose, turkey, quail, pheasant, parrot, finches, hawk, crow, ostrich, emu and cassowary), primate (e.g., prosimian, tarsier, monkey, gibbon, ape), humans, and fish. The term "animal" also includes an individual animal in all stages of development, including embryonic and fetal stages. The term "pig" or "piglet" means an animal of porcine origin, while "sow" refers to a female of reproductive age and capability.

*Abbreviations*: ELISA, enzyme-linked immunosorbant assay; Mab, monoclonal antibody; FITC, Fluorescein isothiocyanate; DAPI: Hoechst stain (blue fluorescent dye); DAPI: 4',6-diamidino-2-phenylindole; GFP: green fluorescent protein; TRITC: Tetramethylrhodamine; Cy5: Cyanine 5.

Described herein are high throughput methods for the determination of the viral titer of a sample containing a virus.

The high throughput methods as described herein comprise the steps of: 1) providing a sample containing a virus; 2) preparing serial dilutions of the sample; 3) infecting host cells with the virus on 96-well plates and incubating the culture; 4) reacting an antigen expressed by the virus in infected cells with an antibody labeled with a fluorescent tag; 5) determining the number of infected cells; and 6) determining the virus titer in the sample.

Conventional plate assays of the virus titer determination that are based on immunofluorescence detection are time consuming and labour intensive. They involve serial dilutions and many duplicates to count the wells (in 96-well plate) with infected cells. The samples to be tested are limited for each session.

In one aspect of the embodiment, the number of infected cells is determined by CellInsight (Thermo Fisher Scientific Inc.) using a multi-well plate. The number of infected cells in each well is read using CellInsight. The multi-well plate may be a 48-well plate, or a 96-well plate.

In another aspect of the embodiment, a straight line is plotted using linear regression between number of fluorescence cells and virus titer using a virus reference. In one aspect, at least one of the serial dilutions of the sample falls within the range of the liner regression line. The number of infected cells per well for the at least one dilution of the sample may preferably fall between $0.1Log_{10}$ and $6.0Log_{10}$.

As described herein the method of the invention allows the detection and monitoring of a virus and infected cells by detecting an antigen expressed by the virus in the host cells until the harvest point. The rapidity with which these data are obtained permits frequent periodic monitoring of the progress of the infection of the cell culture. The culture may then be harvested at a point that gives a high yield of virus suitable for vaccine development and production or for seeding a large batch host cell culture for the ultimate production of virus of sufficient quantity for use in, for example, vaccine development and production.

The seed cultures developed as a result of the use of the methods of the invention can be used to seed large volume fresh cell culture medium, in the order of 5-1 000 liter volumes or more. In this procedure, the seed culture harvested is inoculated into a large-scale culture comprising cell culture medium.

This invention, therefore, provides a high throughput procedure for the in-process monitoring and rapid determination of the useful harvesting point of a cell culture infected with a virus such that an optimum yield of the virus can be obtained. The methods as described herein encompass providing a seed culture of a virus infected host cells and inoculating a batch culture therewith, incubating the culture, removing samples of the cultured cells, preparing serial dilutions of the samples, reacting an antigen expressed by the virus in infected cells with an antibody labeled with a fluorescent tag, determining the number of fluorescence cells, thereby determining the virus titer in the batch culture. It is contemplated that the methods of the invention can be usefully applied for detecting and in-process monitoring of the production of any virus cultured on isolated host cells and for which there are available viral antigen specific antibodies.

In the various embodiments of this method of the invention, the viruses can be any viruses, including DNA viruses, RNA viruses, recombinant viruses, viruses carrying transgenes, baculoviruses expressing antigens, etc. The viruses may include adenovirus, retrovirus, herpes simplex virus, parvovirus, classical swine fever virus, influenza virus, circovirus (including porcine circovirus PCV1 and PCV2), porcine reproductive and respiratory syndrome virus, rotavirus, bovine viral diarrhea virus, porcine epidemic diarrhea virus, sindbis virus, baculovirus, pseudorabies virus, varicella-zoster virus, cytomegalovirus, vesicular stomatitis virus, hepatitis A, B, and C viruses, pox virus, foot-and-mouth disease virus, bluetongue virus, newcastle disease virus, infectious bursal disease virus, Marek's disease virus, infectious laryngotracheitis virus, avian paramyxovirus, westnile virus, nipah virus, hendra virus, African horse sickness virus, canine distemper virus, leukemia virus, calicivirus, Schmallenberg virus, and recombinant viruses thereof.

## EXAMPLES

Example 1 Summary of the current virus titration plate assay

The viruses used in this example were canine parvovirus (CPV) and recombinant pox virus (avian canarypox virus) expressing an equine influenza antigen. The host cell line is canine myeloma cells A72.

### Host cells preparation

A fresh suspension of host cells was prepared in DMEM medium +5% FCS (Fetal Calf Sera) at a concentration of 60 000 cells/ml. 150 μl of this cell suspension was added into each well of a 96-well plate.

### Sample dilutions

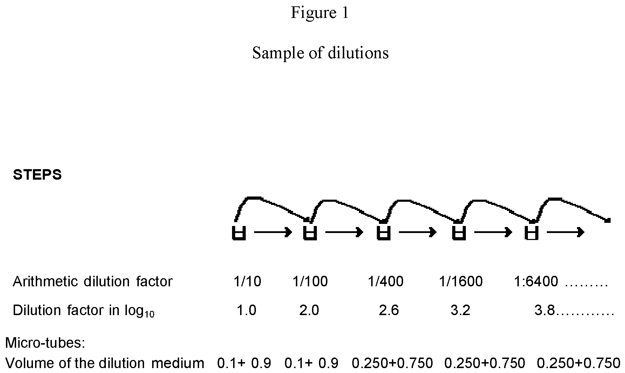

An aliquot was taken from a sample containing a virus and serial dilutions were carried out in micro tubes according to, for example, the table shown in Figure 1. The virus control (virus reference) was diluted following the same procedure.

### Plate design

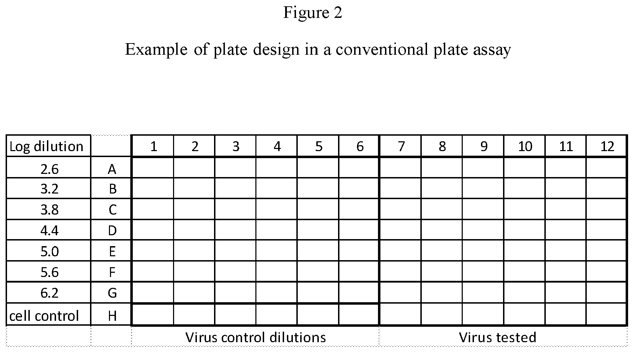

The prepared dilutions that encompass the expected titer were dispensed into the plate as shown in Figure 2. To each well containing 150 μl of the cell suspension, 100 μl of the diluted samples were added. For cell controls (line H) (at least one cell control per session), 100 μl of dilution medium was added to the cell suspension. The number of sample dilutions was 8. The number of virus control dilutions was 7. The number of wells per dilution is 6. In this plate design, for one plate, total of 42 wells were used for virus control (virus reference), 6 wells were used for cell control, and 48 wells were used to assay one sample. In some cases, 144 wells (one and a half 96-well plates) were used to read one sample, and the resulting three results per sample were used to calculate the average reading of the sample.

In this method, to calculate a titer of a sample, one or two 96-well plates are needed to obtain an accurate titer determination.

### Virus infection of host cells in 96-well plates

The plates were incubated for 4 days in an incubator at around 37°C with 5% $CO_2$. At the end of the incubation period, the control wells had to be checked under an inverted microscope for the completion of the cell layer without toxicity and pollution.

### Fixation of plates for immunofluorescence reaction

The plates were fixed with acetone. Briefly, the plates were washed once with PBS[2-] Buffer Saline Phosphate without calcium and magnesium. 100 μl/well of cold acetone 90% (10% of water) was added to the plates and the plates were incubated at -20°C for 30 min. The acetone was discarded and the plates were completely air-dried.

### Immunofluorescence reaction

For the immunofluorescence reaction, 100 μl/well monoclonal antibody (anti-CPV2 for CPV or anti-EIV antigen for the recombinant Canarypox-equine influenza antigen) was added to the plates and the plates were incubated at 37°C for 30 min. The plates then were washed twice using PBS. 100 μl/well anti-mouse FITC was added to the plates and the plates were incubated at 37°C for 30 min. The plates were washed with PBS twice and followed by a rapid wash in neutral water.

### Titer calculation

The plates were read by UV light with filter 488-519λ. For identification, the well is positive if one or more cells are fluorescent.

The calculation of the titer is carried out by angular transformation or according to Karber's method, for which the formula is:

$$T = d + [r/N] \times [n + N/2] + 1$$

T = titer
d = dilution with 100% of wells to show a fluorescent focus
r = dilution ratio
N = number of wells per rate dilution
n = number of wells showing a fluorescence for rate dilution above d

Example 2 Improved high throughput method of virus titration

The viruses used in this example were canine parvovirus (CPV) and recombinant pox virus (avian canarypox virus) expressing an equine influenza antigen. The host cell line is canine myeloma cells A72.

Host cells preparation was carried out as described in Example 1.

### Sample dilution

Sample dilution was carried out as described in Example 1.

### Plate design

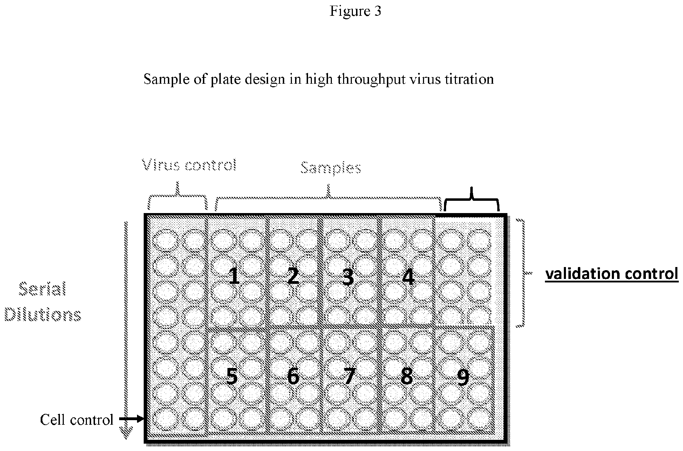

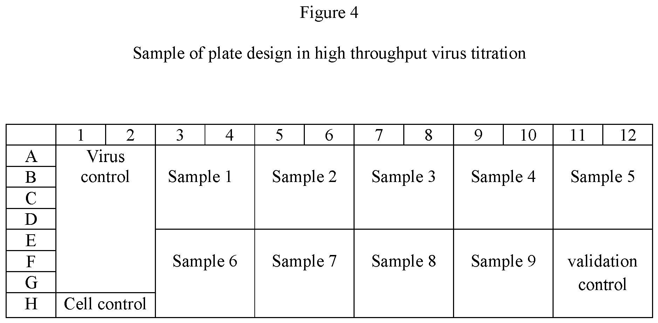

A sample plate is shown in Figures 3 and 4. In these plate designs, the number of sample dilutions was 4. The number of virus control dilutions was 7. The number of wells per dilution is 2. For one plate, total of 14 wells were used for virus control (virus reference), 2 wells were used for cell control, 8 wells were used for validation control per session (each session may contain multiple plates), and 8 wells were used to assay one sample for a total of 9 samples. Virus reference was used to plot the linear regression line and to calculate the titer of the unknown sample. Cells control were wells without any virus to check that the session worked well. Validation control contains a virus (other than the virus reference) with known titer. Validation control was titrated on each plate and its titer was calculated and the results were included in the chart control. Validation control provides quality control of the session and ensures that the session worked well.

Virus infection of host cells in 96-well plates, Fixation of plates for immunofluorescence reaction, and Immunofluorescence reaction were carried out as described in Example 1.

Titer calculation

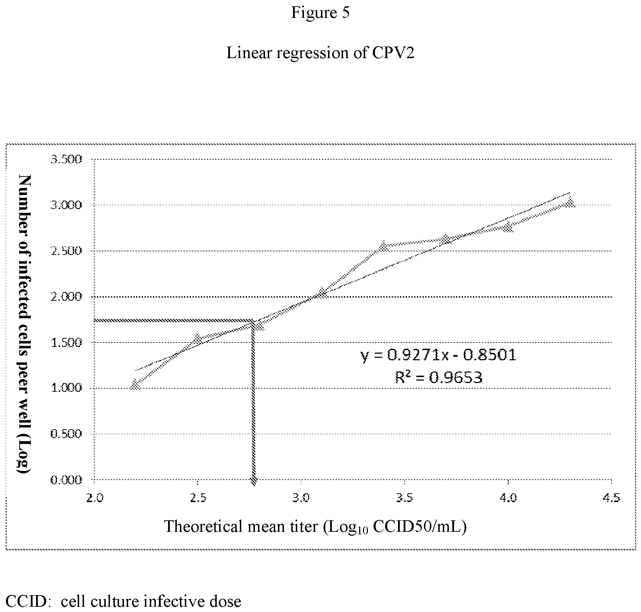

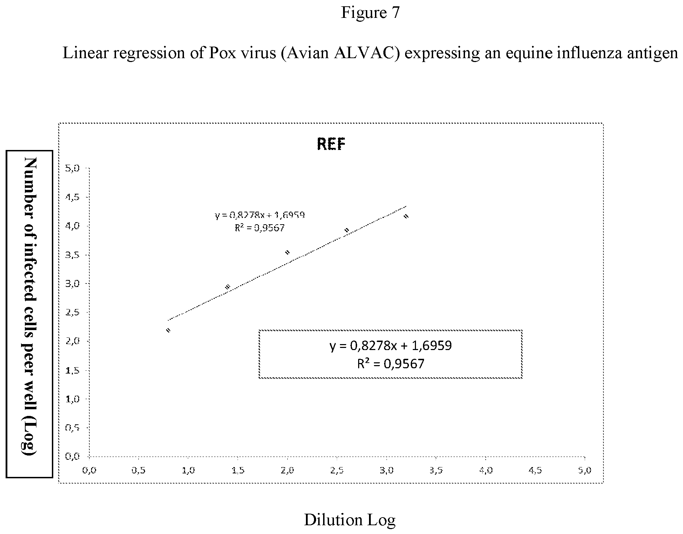

The number of infected cells per well was read using CellInsight. A straight line is plotted using linear regression between number of fluorescence cells per well and virus titer using the virus control (virus reference) as shown in Figure 5 (CPV) and Figure 7 (recombinant pox virus). The virus titer of a sample is then calculated using the straight line (linear regression of reference virus) based on the reading of the infected cells per well of that sample and its dilution factor.

Results

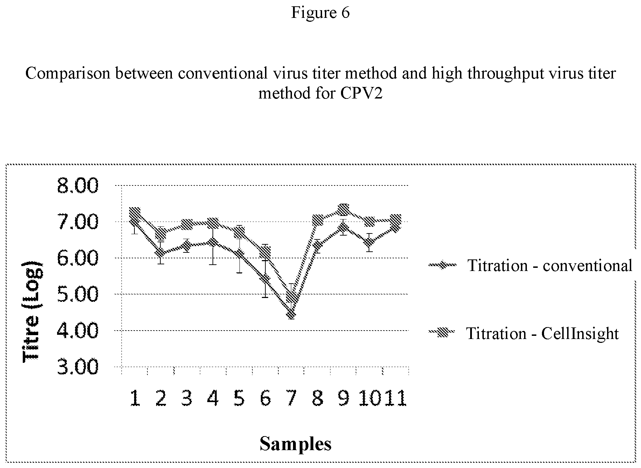

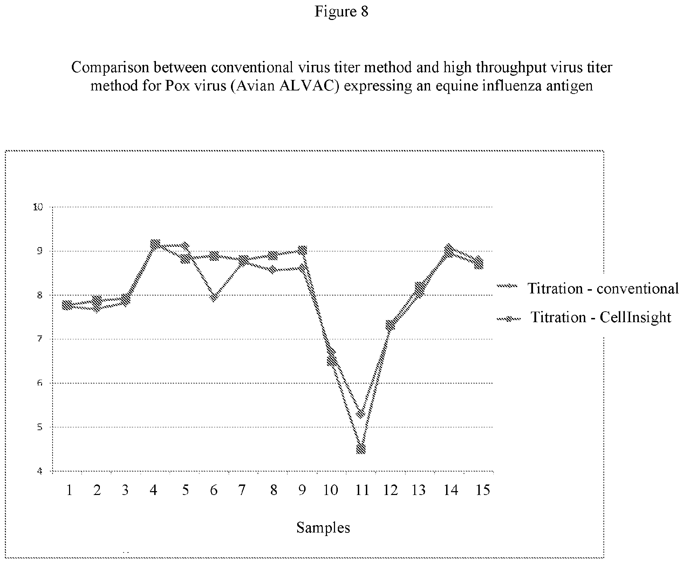

The virus titer calculated using the improved method was compared to the virus titer calculated using the conventional method. Figure 6 shows the results for CPV. Figure 8 shows the results for recombinant pox virus. The results demonstrated that the high throughput method which determines the infected cells collates well with the conventional virus titer determination method (conventional plate assay) which counts the wells containing infected cells (fluorescence tagged cells).

Discussion

This high throughput method provides the advantage of reducing the number of dilutions and duplicates that is necessary to accurately calculate the virus titer, and therefore the increase of number of samples to be titrated on one plate.

**Claims**

**1.** A method of virus quantification comprising the steps of:

1) providing a sample containing a virus;

2) preparing serial dilutions of the sample;

3) infecting host cells with the virus and incubating the culture;

4) reacting an antigen expressed by the virus in infected cells with an antibody labeled with a fluorescent tag;

5) determining the number of infected cells by using an image cytometer wherein the image cytometer is a component of a single benchtop instrument which combines the functions of a digital microscope, said image cytometer and a cell counter, preferably wherein said image cytometer is capable of making individual cell, subcellular and multi-cellular measurements and capturing cellular and sub-cellular image data; and

6) determining the virus titer in the sample, wherein a straight line is plotted using linear regression between number of fluorescence tagged cells and virus titer using a virus reference.

**2.** The method of claim 1, wherein the method is carried out in a multi-well plate.

**3.** The method of claim 2, wherein the multi-well plate is a 96-well plate.

**4.** The method of claim 2 wherein the multi-well plate is a 48-well plate.

**5.** The method of any one of claims 2 to 4, wherein the number of infected cells is determined by reading the fluorescence tagged cells in each well.

**6.** The method of claim 5, wherein the fluorescent tag is selected from the group consisting of FITC, Hoechst stain, DAPI, GFP, TRITC and Cy5.

**7.** The method of any one of claims 1 to 6, wherein at least one of the serial dilutions falls within the range of the liner regression line of the reference virus.

**8.** The method of any one of claims 1 to 7 wherein the viruses are DNA viruses or RNA viruses.

**9.** The method of claim 8 wherein the viruses are adenoviruses, retroviruses, herpes simplex viruses, parvoviruses, classical swine fever viruses, influenza viruses, circoviruses (including porcine circoviruses PCV1 and PCV2), porcine reproductive and respiratory syndrome viruses, rotaviruses, bovine viral diarrhea viruses, porcine epidemic diarrhea viruses, sindbis viruses, baculoviruses, pseudorabies viruses, varicella-zoster viruses, cytomegaloviruses, vesicular stomatitis viruses, hepatitis A, B, and C viruses, pox viruses, foot-and-mouth disease viruses, bluetongue viruses, newcastle disease viruses, infectious bursal disease viruses, Marek's disease viruses, infectious laryngotracheitis viruses, avian paramyxoviruses, westnile viruses, nipah viruses, hendra viruses, African horse sickness viruses, canine distemper viruses, leukemia viruses, caliciviruses or Schmallenberg viruses.

10. The method of claim 9 wherein the viruses are recombinant viruses.

11. Use of the method as defined in any one of claims 2 to 10, in a high throughput procedure for the in-process monitoring and rapid determination of the useful harvesting point of a cell culture infected with a virus such that an optimum yield of the virus can be obtained.

**Patentansprüche**

1. Verfahren zur Virus-Quantifizierung, umfassend die Schritte:

   1) Bereitstellen einer Probe, die ein Virus enthält;
   2) Herstellen von Verdünnungsreihen der Probe;
   3) Infizieren von Wirtszellen mit dem Virus und Inkubieren der Kultur;
   4) In-Reaktion-bringen eines Antigens, das von dem Virus in infizierten Zellen exprimiert wird, mit einem Antikörper, der mit einer Fluoreszenzmarkierung markiert ist;
   5) Bestimmen der Anzahl infizierter Zellen durch die Verwendung eines Bildcytometers, wobei das Bildcytometer ein Bestandteil eines einzelnen Benchtop-Geräts ist, das die Funktionen eines digitalen Mikroskops, des Bildcytometers und eines Zellzählers kombiniert, bevorzugt wobei das Bildcytometer in der Lage ist, Messungen von einzelnen Zellen, subzelluläre und multizelluläre Messungen zu machen und zelluläre und subzelluläre Bilddaten zu erfassen; und
   6) Bestimmen des Virustiters in der Probe, wobei unter Verwendung linearer Regression eine gerade Linie zwischen der Anzahl von Zellen mit Fluoreszenzmarkierung und Virustiter aufgetragen wird unter Verwendung einer Virusreferenz.

2. Verfahren nach Anspruch 1, wobei das Verfahren in einer Multi-Well-Platte durchgeführt wird.

3. Verfahren nach Anspruch 2, wobei die Multi-Well-Platte eine 96-Well-Platte ist.

4. Verfahren nach Anspruch 2, wobei die Multi-Well-Platte eine 48-Well-Platte ist.

5. Verfahren nach einem der Ansprüche 2 bis 4, wobei die Anzahl an infizierten Zellen durch Ablesen der Zellen mit Fluoreszenzmarkierung in jedem Well bestimmt wird.

6. Verfahren nach Anspruch 5, wobei die Fluoreszenzmarkierung ausgewählt ist aus der Gruppe bestehend aus FITC, Hoechst-Farbstoff, DAPI, GFP, TRITC und Cy5.

7. Verfahren nach einem der Ansprüche 1 bis 6, wobei mindestens eine der Serienverdünnungen in den Bereich der linearen Regressionslinie des Referenzvirus fällt.

8. Verfahren nach einem der Ansprüche 1 bis 7, wobei die Viren DNA-Viren oder RNA-Viren sind.

9. Verfahren nach Anspruch 8, wobei die Viren Adenoviren, Retroviren, HerpesSimplex-Viren, Parvoviren, klassische Schweinepestviren, Influenzaviren, Circoviren (einschließlich porcinen Circoviren PCV1 und PCV2), Porcine Reproductive and Respiratory Syndrome-Viren, Rotaviren, bovine Virusdiarrhö-Viren, porcine epidemische Diarrhöviren, Sindbis-Viren, Baculoviren, Pseudorabies-Viren, Varicella-Zoster-Viren, Cytomegaloviren, vesikuläre Stomatitis-Viren, Hepatitis A-, B- und C-Viren, Pockenviren, Maul- und Klauenseuche-Viren, Blauzungenviren, Newcastle-Krankheit-Viren, infektiöse Bursitis-Viren, Marek-Krankheit-Viren, infektiöse Laryngotracheitis-Viren, Vogel-Paramyxoviren, West-Nil-Viren, Nipah-Viren, Hendra-Viren, afrikanische Pferdepest-Viren, canine Staupeviren, Leukämieviren, Caliciviren oder Schmallenberg-Viren sind.

10. Verfahren nach Anspruch 9, wobei die Viren rekombinante Viren sind.

11. Verwendung des Verfahrens wie in einem der Ansprüche 2 bis 10 definiert, in einem Hochdurchsatzverfahren zur In-Prozess-Überwachung und schnellen Bestimmung des nützlichen Erntezeitpunkts einer Zellkultur, die mit einem Virus infiziert ist, so dass ein optimaler Ertrag des Virus erhalten werden kann.

**Revendications**

1. Procédé de quantification de virus comprenant les étapes de:

   1) fournir un échantillon contenant un virus;
   2) préparer des dilutions en série de l'échantillon;
   3) infecter des cellules hôtes avec le virus et incuber la culture;
   4) faire réagir un antigène exprimé par le virus dans des cellules infectées avec un anticorps marqué avec un marqueur fluorescent;

5) déterminer le nombre de cellules infectées en utilisant un cytomètre d'image dans lequel le cytomètre d'image est un composant d'un instrument de paillasse unique qui combine les fonctions d'un microscope numérique, dudit cytomètre d'image et d'un compteur de cellules, de préférence dans lequel ledit cytomètre d'image est capable de faire des mesures de cellules individuelles, sub-cellulaires et multicellulaires et de capturer des données d'images cellulaires et sub-cellulaires; et

6) déterminer le titre viral dans l'échantillon, dans lequel une ligne droite est tracée en utilisant une régression linéaire entre le nombre de cellules marquées par fluorescence et le titre viral en utilisant une référence virale.

2. Procédé selon la revendication 1, dans lequel le procédé est réalisé dans une plaque à puits multiples.

3. Procédé selon la revendication 2, dans lequel la plaque à puits multiples est une plaque à 96 puits.

4. Procédé selon la revendication 2, dans lequel la plaque à puits multiples est une plaque à 48 puits.

5. Procédé selon l'une quelconque des revendications 2 à 4, dans lequel le nombre de cellules infectées est déterminé en lisant les cellules marquées par fluorescence dans chaque puits.

6. Procédé selon la revendication 5, dans lequel le marqueur fluorescent est choisi dans le groupe consistant en FITC, colorant Hoechst, DAPI, GFP, TRITC et Cy5.

7. Procédé selon l'une quelconque des revendications 1 à 6, dans lequel au moins l'une des dilutions en série est située dans la plage de la ligne de régression linéaire du virus de référence.

8. Procédé selon l'une quelconque des revendications 1 à 7, dans lequel les virus sont des virus à ADN ou des virus à ARN.

9. Procédé selon la revendication 8, dans lequel les virus sont des adénovirus, des rétrovirus, des virus de l'herpès simplex, des parvovirus, des virus de la peste porcine classique, des virus de la grippe, des circovirus (y compris des circovirus porcins PCV1 et PCV2), des virus du syndrome reproducteur et respiratoire porcin, des rotavirus, des virus de la diarrhée virale bovine, des virus de la diarrhée épidémique porcine, des virus sindbis, des baculovirus, des virus pseudorabiques, des virus varicelle-zona, des cytomégalovirus, des virus de la stomatite vésiculeuse, des virus de l'hépatite A, B et C, des virus de la variole, des virus de la fièvre aphteuse, des virus de la fièvre catarrhale, des virus de la maladie de Newcastle, des virus de la maladie de bursite infectieuse, des virus de la maladie de Marek, des virus de la laryngotrachéite infectieuse, des paramyxovirus aviaires, des virus du Nil occidental, des virus nipah, des virus hendra, des virus de la peste équine africaine, des virus de la maladie de Carré canine, des virus de la leucémie, des calicivirus ou des virus de Schmallenberg.

10. Procédé selon la revendication 9, dans lequel les virus sont des virus recombinants.

11. Utilisation du procédé tel que défini dans l'une quelconque des revendications 2 à 10, dans une procédure à haut débit pour la surveillance en cours de processus et la détermination rapide du point de récolte utile d'une culture cellulaire infectée par un virus de sorte qu'un rendement optimal du virus peut être obtenu.

Figure 1

Sample of dilutions

**STEPS**

| | | | | | |
|---|---|---|---|---|---|
| Arithmetic dilution factor | 1/10 | 1/100 | 1/400 | 1/1600 | 1:6400 ......... |
| Dilution factor in $\log_{10}$ | 1.0 | 2.0 | 2.6 | 3.2 | 3.8............ |

Micro-tubes:

| Volume of the dilution medium | 0.1+ 0.9 | 0.1+ 0.9 | 0.250+0.750 | 0.250+0.750 | 0.250+0.750 |
|---|---|---|---|---|---|

Figure 2

Example of plate design in a conventional plate assay

| Log dilution | | 1 | 2 | 3 | 4 | 5 | 6 | 7 | 8 | 9 | 10 | 11 | 12 |
|---|---|---|---|---|---|---|---|---|---|---|---|---|---|
| 2.6 | A | | | | | | | | | | | | |
| 3.2 | B | | | | | | | | | | | | |
| 3.8 | C | | | | | | | | | | | | |
| 4.4 | D | | | | | | | | | | | | |
| 5.0 | E | | | | | | | | | | | | |
| 5.6 | F | | | | | | | | | | | | |
| 6.2 | G | | | | | | | | | | | | |
| cell control | H | | | | | | | | | | | | |
| | | Virus control dilutions | | | | | | Virus tested | | | | | |

Figure 3

Sample of plate design in high throughput virus titration

Figure 4

Sample of plate design in high throughput virus titration

| | | 1 | 2 | 3 | 4 | 5 | 6 | 7 | 8 | 9 | 10 | 11 | 12 |
|---|---|---|---|---|---|---|---|---|---|---|---|---|---|
| A | | Virus control | | Sample 1 | | Sample 2 | | Sample 3 | | Sample 4 | | Sample 5 | |
| B | | | | | | | | | | | | | |
| C | | | | | | | | | | | | | |
| D | | | | | | | | | | | | | |
| E | | | | Sample 6 | | Sample 7 | | Sample 8 | | Sample 9 | | validation control | |
| F | | | | | | | | | | | | | |
| G | | | | | | | | | | | | | |
| H | | Cell control | | | | | | | | | | | |

Figure 5

Linear regression of CPV2

$$y = 0.9271x - 0.8501$$
$$R^2 = 0.9653$$

CCID:  cell culture infective dose

Figure 6

Comparison between conventional virus titer method and high throughput virus titer
method for CPV2

Figure 7

Linear regression of Pox virus (Avian ALVAC) expressing an equine influenza antigen

Figure 8

Comparison between conventional virus titer method and high throughput virus titer method for Pox virus (Avian ALVAC) expressing an equine influenza antigen

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62196886
- US 6248514 B
- US 7476507 B
- WO 9801582 A

**Non-patent literature cited in the description**

- **ERIKA HAMMARLUND et al.** A Flow Cytometry-Based Assay for Quantifying Non-Plaque Forming Strains of Yellow Fever Virus. *PLOS ONE,* 19 September 2012, vol. 7 (9), e41707